# EUROPEAN PATENT APPLICATION

(11) **EP 2 712 591 A1**
(43) Date of publication of application: **02.04.2014**
(21) Application number: 13186641.0
(22) Date of filing: 30.09.2013
(51) Int. Cl.: A61F 9/007

(54) **Ophthalmic surgical tip, ophthalmic surgical handpiece including the tip, and ophthalmic surgical apparatus**

(30) Priority: 01.10.2012 JP 2012219362; 27.09.2013 JP 2013201741
(71) Applicant: Nidek Co., Ltd., Aichi 443-0038 (JP)
(72) Inventor: Oda, Hideo, Gamagori-shi, Aichi 4430038 (JP); Kato, Hisatoshi, Gamagori-shi, Aichi 4430038 (JP); Ohashi, Yuya, Gamagori-shi, Aichi 4430038 (JP)
(74) Representative: Prüfer & Partner GbR European Patent Attorneys

(57) **Abstract**

An ophthalmic surgical tip (60) which will be attached to an ophthalmic surgical handpiece (10) including an ultrasonic transducer (3) is configured to fracture a lens nucleus by utilizing torsional vibration from the ultrasonic transducer, the tip including a small tube internally formed with an aspiration passage (66), and the tip being arranged to aspirate and remove fractured tissues through the aspiration passage. The tip includes a hollow shaft (65) in which the small tube is formed, and a head part (61) formed on a leading end side of the shaft, the head part including: a leading end portion (62) formed in a rounded shape at a leading end of the head part; a fracturing portion (63) formed behind the leading end portion and having a protrusion to fracture a lens nucleus by torsional vibration; and an aspiration port (64) connected to the small tube.

## Description

### BACKGROUND OF THE INVENTION

### Field of the Invention

The present invention relates to an ophthalmic surgical tip to be used in emulsification and removal surgery (phacoemulsification and aspiration) of a lens of a patient's eye and others, a handpiece including this ophthalmic surgical tip, and an ophthalmic surgical apparatus.

### Related Art

A cataract surgery uses an apparatus for fracturing and removing a lens by utilizing ultrasonic vibration (see e.g. Patent Document 1). A cataract surgical apparatus used in the cataract surgery is arranged such that ultrasonic vibration is transmitted to a tubular ophthalmic surgical ultrasonic tip (hereinafter, referred to as a US tip) attached to a leading end of a handpiece having an ultrasonic transducer, thereby fracturing and emulsifying a lens nucleus, and removing the fractured and emulsified lens nucleus by aspiration through an aspiration port of the tip. In a case where residual materials in a capsule, for example, fragments of the lens nucleus, cortical substances adhering to the capsule, and others are to be removed after removal of the lens nucleus, the handpiece is replaced with another handpiece. This handpiece is not required to fracture or chop and thus is configured to make only aspiration. In the handpiece, an irrigation aspiration tip (hereinafter, simply referred to as an IA tip) having a leading end shaped to hardly damage the capsule is attached to remove residual materials by aspiration and abrade the capsule and others. As above, the cataract surgery is performed using two types of tips.

There is recently proposed a technique that utilizes torsional vibration in a turning (torsional) direction of the US tip about its long axis as compared with a conventional liner motion (vertical vibration in a long axis direction) of ultrasonic vibration of the US tip (see for example Patent Document 2). A tip used for such torsional vibration is formed with a leading end portion bent to enhance a fracturing efficiency to a lens nucleus.

In recent years, furthermore, there is proposed a technique to fracture and cut a lens nucleus by use of a photodisruptive action of an extremely-short pulse laser such as femtosecond laser (see for example Patent Document 3). With the use of this apparatus, it is expected that the efficiency of cataract surgery is enhanced and the accuracy is improved.

### Related Art Documents

### Patent Documents

- Patent Document 1:: JP 2011-212350A
- Patent Document 2:: US 6077285
- Patent Document 3:: US Application Pub. No. 2009/0177189

### SUMMARY OF INVENTION

### Problems to be Solved by the Invention

As explained above, the US tip and the IA tip are used for different purposes. The surgery thus requires a work to replace a handpiece for the US tip and a handpiece for the IA tip. This takes time and efforts.

The present invention has been made to solve the above problems and has a purpose to provide an ophthalmic surgical tip capable of saving time and efforts for replacing handpieces, an ophthalmic surgical handpiece including this tip, and an ophthalmic surgical apparatus.

### Means of Solving the Problems

To achieve the above purpose, one aspect of the invention provides an ophthalmic surgical tip which will be attached to an ophthalmic surgical handpiece including an ultrasonic transducer, the tip being configured to fracture a lens nucleus by utilizing torsional vibration from the ultrasonic transducer, the tip including a small tube internally formed with an aspiration passage, and the tip being arranged to aspirate and remove fractured tissues through the aspiration passage, characterized by including: a hollow shaft in which the small tube is formed; and a head part formed on a leading end side of the shaft, the head part including: a leading end portion formed in a rounded shape at a leading end of the head part; a fracturing portion formed behind the leading end portion and having a protrusion to fracture a lens nucleus by torsional vibration; and an aspiration port connected to the small tube.

Further, another aspect of the invention provides an ophthalmic surgical handpiece including the above ophthalmic surgical tip.

Still further, another aspect of the invention provides an ophthalmic surgical apparatus including the above ophthalmic surgical handpiece.

Further developments of the present invention are given in the dependent claims.

### Effects of the Invention

According to the invention, it is possible to save time and efforts for replacing tips and handpieces during surgery.

### BRIEF DESCRIPTION OF THE DRAWINGS

FIG. 1 is a configuration view of an ophthalmic surgical apparatus;
FIG. 2 is a schematic configuration view of an ophthalmic laser surgical apparatus;
FIG. 3 is an enlarged perspective view of a leading end portion of a tip;
FIG. 4 is a side view of the leading end portion of the tip; and
FIG. 5 is a cross sectional view taken along a line A-A in FIG. 4.

### DESCRIPTION OF EMBODIMENTS

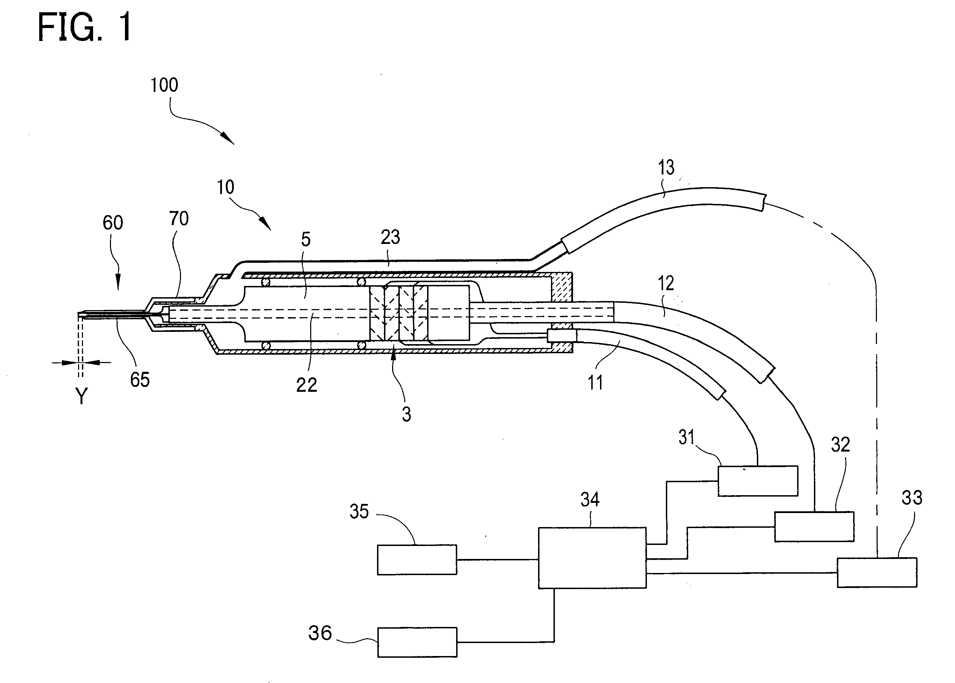

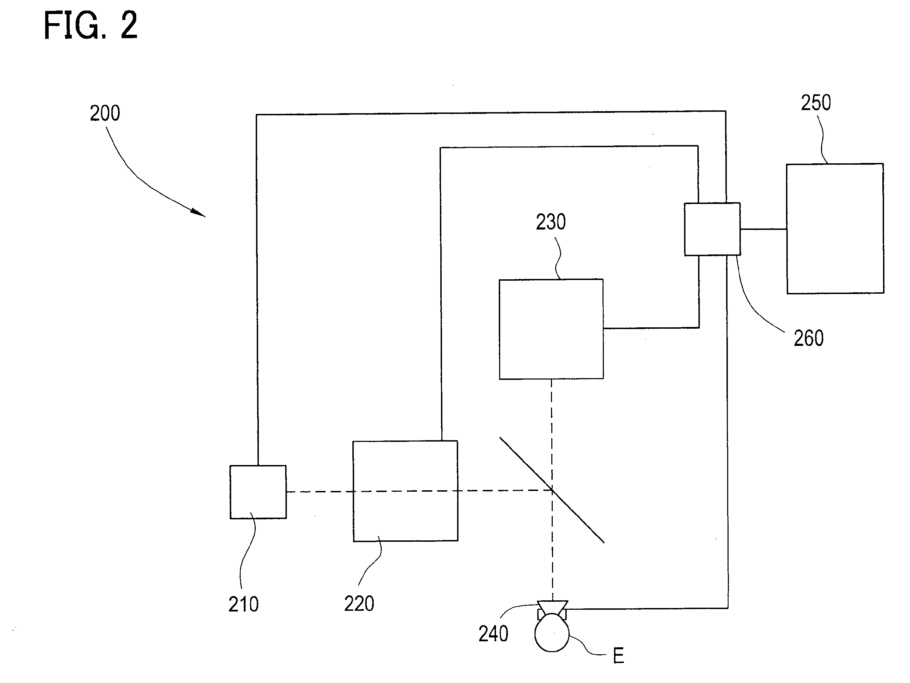

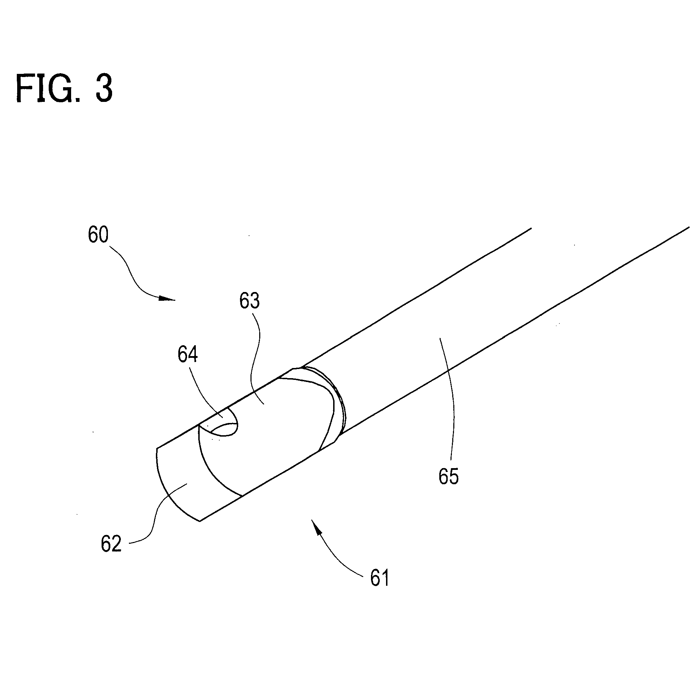

A detailed description of an embodiment of the present invention will now be given referring to the accompanying drawings. FIG. 1 is a schematic configuration view of an ophthalmic surgical handpiece including an ophthalmic surgical tip attached at a leading end and a control system connected to the handpiece. FIG. 2 is a schematic configuration view of an ophthalmic laser surgical apparatus arranged to cut and fracture a lens and others by laser. FIGs. 3 to 5 are views to explain the shape of the ophthalmic surgical tip in the embodiment.

An ophthalmic surgical apparatus (an ophthalmic ultrasonic surgical apparatus) 100 shown in FIG. 1 includes a handpiece 10, a drive unit 31, an aspiration unit 32, an irrigation unit 33, a controller 34, a setting part 35, and an operation unit 36. In the present embodiment, those drive unit 31, aspiration unit 32, irrigation unit 33, controller 34, and setting part 35 are housed in a casing not illustrated. The handpiece 10 is connected to the casing through a cable 11, an aspiration tube 12, and an irrigation tube 13. The operation unit 36 is connected to the casing through a cable for communicating control signals and others.

The handpiece 10 which will be grasped by an operator includes an ultrasonic transducer 3, a horn 5, a tip (ultrasonic tip, ophthalmic surgical tip) 60, and a sleeve 70. In the handpiece 10, there are formed an aspiration passage 22 and an irrigation passage 23.

The ultrasonic transducer 3 constitutes a vibration system in cooperation with the horn 5 and the tip 60 to generate ultrasonic vibration at resonant frequency from energy (applied voltage) transmitted from the drive unit 31 through the cable 11. The ultrasonic transducer 3 in the present embodiment is composed of a combination of two vibration elements different in vibration direction. The ultrasonic transducer 3 is formed, at the center thereof, with a hole through which the horn 5 extends. The ultrasonic transducer 3 is pressurized by a nut not illustrated.

The horn 5 is a hollow tubular component. A hollow section of the horn 5 forms a part of the aspiration passage 22. The tip 60 is fixed to a leading end of the horn 5. Herein, the tip 60 is secured to the horn 5 with screws. Accordingly, the ultrasonic transducer 3, horn 5, and tip 60 are caused to integrally resonate, so that the tip 60 is also vibrated.

The tip 60 is a hollow tubular component (a small tube). The tip 60 includes a head part 61, a shaft 65, and an aspiration passage 66. The aspiration passage 66 is connected with the horn 5 and hence to the aspiration passage 22.

The sleeve 70 for irrigation attached to the leading end of the handpiece 10 includes a hollow portion to supply irrigation fluid into an eye. The sleeve 70 covers the shaft 65 of the tip 60 (see a length Y in FIG. 1) while a leading end portion of the tip 60 is located outside by about 1 mm. The sleeve 70 is made of flexible material and, for example, molded of silicone. The sleeve 70 is provided, at its leading end, with an outlet (opening) to supply the irrigation fluid into the eye. In the handpiece 10 in which the sleeve 70 is mounted, the irrigation passage 23 is provided to deliver the irrigation fluid into the sleeve 70. A rear end of the irrigation passage 23 is connected to the irrigation tube 13. The outer diameter of a part of the sleeve 70 covering the tip shaft is set to 3.0 mm or less, preferably, 2 mm or less. This is because the sleeve 70 is designed to enable an incision to be formed as small as possible in an eyeball.

The cable 11 connects the ultrasonic transducer 3 and the drive unit 31. The cable 11 is a conductive wire to supply electric power, control signals, and others. The aspiration tube 12 connects the aspiration passage 22 (66) and the aspiration unit 32. The irrigation tube 13 connects the irrigation passage 23 and the irrigation unit 33. Those aspiration tube 12 and irrigation tube 13 are hollow flexible tubes.

The controller 34 for overall controlling the whole apparatus is connected to the drive unit 31, aspiration unit 32, irrigation unit 33, setting part 35, and operation unit 36. The controller 34 controls each unit based on set values set on the setting part 35. The drive unit 31 includes a power supply, a control circuit, and others and serves to vibrate (activate) the ultrasonic transducer 3. The aspiration unit 32 includes an aspiration pump and others and serves to apply aspiration pressure to the tip 60 (the leading end thereof). By the aspiration unit 32, tissues of a fractured or chopped lens are aspirated and removed through the aspiration passage 22 and others. The aspirated waste is discharged out into a waste bag (not illustrated). The irrigation unit 33 includes an irrigation bottle, an irrigation valve, and others and serves to supply the irrigation fluid from the sleeve 70 (the leading end thereof). By the irrigation unit 33, the irrigation fluid is supplied at a set irrigation pressure through the irrigation passage 23.

The setting part 35 serves to set (input) surgical conditions. The setting part 35 is for example a touch panel display provided in the casing of the apparatus. Accordingly, the setting part 35 also serves as a display part for the surgical conditions and others. This allows an operator to set, at the touch, aspiration pressure (adjustment parameters thereof), irrigation pressure, ultrasonic vibration output, operational conditions (parameters of an operation mode of a handpiece, etc.) by the operation unit 36, and others. One example of the operation unit 36 is a foot pedal to transmit a control signal to the controller 34 in correspondence with a depression amount (an operation amount) by the operator. The handpiece 10 is configured to be switched between at least two operation modes according to the operation amount by which the operation unit 36 is operated. The operation modes include an ultrasonic vibration mode of performing irrigation aspiration while ultrasonically vibrating the tip 60 and an irrigation aspiration mode of performing only irrigation aspiration without ultrasonically vibrating the tip 60. In each mode, the power of ultrasonic vibration, aspiration amount, and others are increased and decreased in proportion to the operation amount of the operation unit 36.

Herein, the ultrasonic vibration (oscillation) of a tip in the present embodiment will be explained. The ultrasonic transducer 3 functions to torsionally vibrate the tip 60. In the present embodiment, torsional vibration represents rotary reciprocating motion (turning motion) of the tip 60 about the long axis. The vibration directions of the two elements constituting the ultrasonic transducer 3 are set different, for example, one is set to a lateral direction and the other is set to a long axis direction. Accordingly, the ultrasonic transducer 3 torsionally vibrates (reciprocating turning vibration) with required vibration amplitude. The leading end of the tip 60 is thus torsionally vibrated. For the liner vibration and torsional vibration of the tip 60 by use of the vibration elements that make ultrasonic oscillation, the technique disclosed in US 6077285 is available.

For the configuration of the handpiece to torsionally vibrate the tip 60 in the present embodiment, the following is also available.

The handpiece 10 includes the ultrasonic transducer 3, horn 5, and sleeve 70. The ultrasonic transducer 3 is a piezoelectric element that makes ultrasonic vibration. The ultrasonic transducer 3 is oriented such that the vibration direction corresponds to a longitudinal direction (long axis direction). The horn 5 is formed with a plurality of slits (or grooves) circumferentially. The slits serve to generate torsion in the horn in response to longitudinal vibration of the horn 5. The slits are formed at such a size and an interval as to generate torsion in the horn 5. The slits are formed at an angle with the long axis (i.e., obliquely). The leading end of the horn 5 is attached with the tip 60. The sleeve 70 is identical in configuration to that of the aforementioned handpiece 10. The drive unit 31 for driving the ultrasonic transducer 3 is configured to enable vibration of the ultrasonic transducer by switching between at least two ultrasonic vibrations different in frequency.

When the ultrasonic transducer 3 is vibrated in accordance with a signal from the drive unit 31, the horn 5 is vibrated along the long axis direction. In association of vibration of the horn 5, the tip 60 is vibrated along the long axis direction (vertically vibrated). When the drive unit 31 transmits a different signal from the above to the ultrasonic transducer 3, the ultrasonic transducer 3 vibrates at a different frequency from the above frequency. Upon receipt of the vertical vibration of a different frequency from the above, the horn 5 produces torsional vibration. The positional interval, angle, and others of the slits are determined to convert the vertical vibration of a vertical specific frequency to torsional vibration. Thus, the horn 5 produces torsional vibration and the tip 60 is also torsionally vibrated.

For the handpiece for torsionally vibrating a tip, a technique disclosed in EP Application Pub. No. 1103238.

The above explanation shows the configuration of switching between the vertical vibration and the torsional vibration of the tip, but the invention is not limited thereto. A configuration of making only torsional vibration may be adopted.

The configuration of an ophthalmic laser surgical apparatus will be explained below. FIG. 2 is a block diagram showing a simplified configuration. In the present embodiment, an ophthalmic laser surgical apparatus 200 is used as a separate apparatus from the ophthalmic surgical apparatus 100. The ophthalmic laser surgical apparatus 200 includes a laser source 210, a laser irradiation unit 220, an observation unit 230, an eyeball fixing unit 240, an operation unit 250, and a controller 260.

The laser source 210 is a light source to emit a laser beam at laser spot (focus point) to induce optical breakdown. As the laser source 210, an ultrashort pulse laser source is used, for example, a femtosecond laser source. The laser irradiation unit (an optical system) 220 includes a light concentrating optical system for forming a laser spot in the tissues (on the tissues) of a patient's eye E and a movement optical system for moving the laser spot in three dimensions. The light concentrating optical system may include for example an objective lens and others. The moving optical system may include for example two galvano mirrors serving as a two-dimensional scanner (XY scanner), a beam expander as a Z scanner.

The observation unit (optical system) 230 is a unit for photographing an anterior segment image and a tomographic image of the patient's eye E. The observation unit 230 includes for example an optical coherence tomography imaging unit (OCT unit) and an anterior segment imaging unit. The eyeball fixing unit 240 serves to restrain eyeball motion of the patient's eye E during laser irradiation. The eyeball fixing unit 240 includes a suction ring for adsorbing the eyeball and a contact glass (an interface unit) for reducing refractive power of a cornea and others.

The operation unit 250 is a unit for setting and operating the apparatus 200. The operation unit 250 includes a touch panel display serving as setting and inputting means and a foot switch serving as trigger input means. The operator sets surgery conditions on the display, makes alignment by using the anterior segment image and the tomographic image, and others displayed on the display, and sets a laser irradiation area, and others. The controller 260 overall controls the whole apparatus. The controller 260 may be arranged to set the surgery conditions based on the data from an external device.

By use of the above ophthalmic laser surgical apparatus 200, the laser spot is three-dimensionally moved in the lens of the patient's eye E, thereby fracturing or cutting the lens nucleus into fragments having a shape easy to be aspirated and removed by the tip 60, for example, a cube of 0.3 mm per side. This can simplify the work of fracturing the lens nucleus by the tip 60.

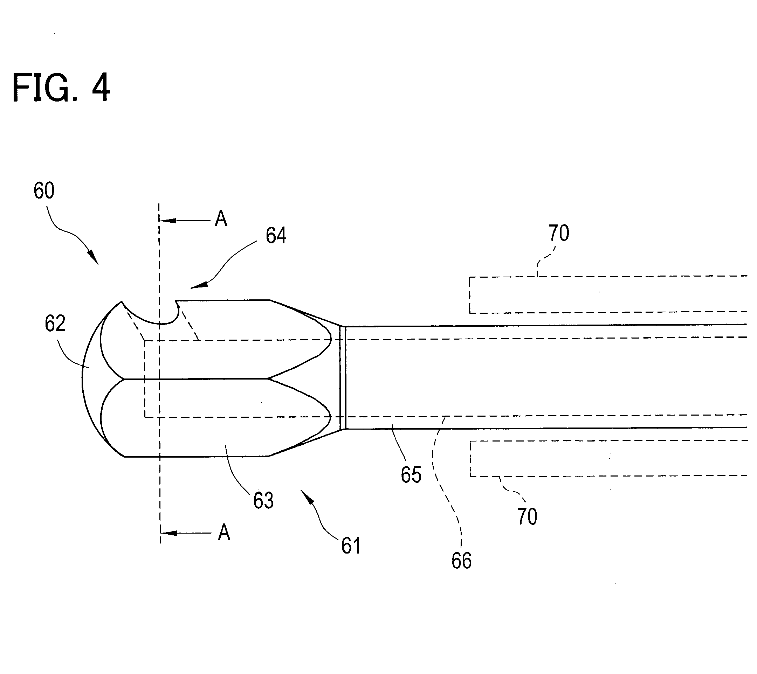

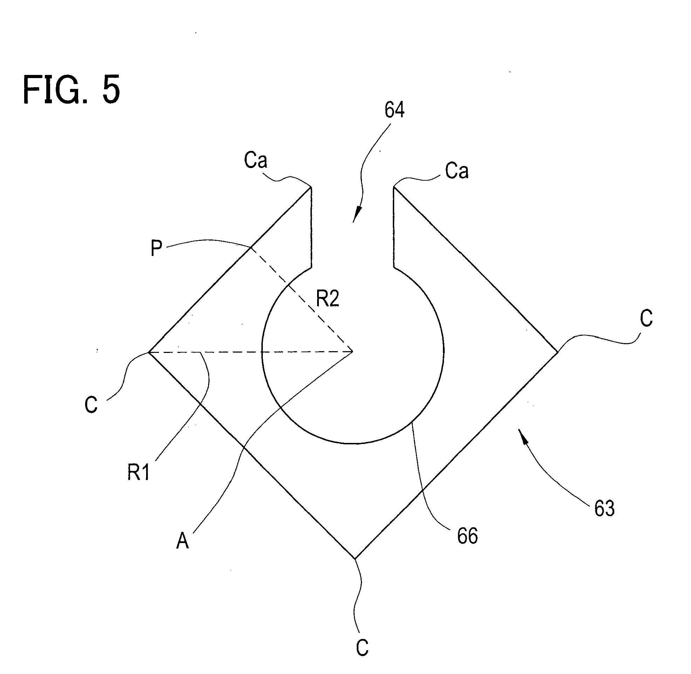

Next, the tip 60 for ophthalmic surgery in the embodiment of the present invention will be explained. FIG. 3 is an enlarged perspective view of the leading end portion of the tip 60. FIG. 4 is a side view of the leading end portion of the tip 60. FIG. 5 is a cross sectional view taken along a line A-A in FIG. 4. In FIG. 4, the position of the sleeve 70 is indicated by a dotted line. In the figures, the side facing the posterior capsule of a lens is in an upward direction.

The shaft 65 of the tip 60 is a hollow small pipe extending in a columnar shape. The proximal end portion of the shaft 65 is fixed to the horn 5. The leading end of the shaft 65 is formed with the head part 61. In the inside (internal space) of the shaft 65, the aspiration passage 66 is formed. The tissues and the fluid aspirated through the head part 61 are delivered to the aspiration unit 32 through the aspiration passage 66. The outer diameter of the shaft 65 is determined to be smaller than the inner diameter of the sleeve 70 to generate a space or clearance (an irrigation passage) between the sleeve 70 and the shaft 65. To be concrete, the outer diameter of the shaft 65 is determined to be 2.0 mm or less, preferably, 1.0 mm or less, and set to 0.9 mm in the present embodiment. The shaft 65 is desired to have a thin wall thickness to ensure the aspiration passage 66. The inner diameter of the shaft 65 in the present embodiment is set to 0.7 mm.

The head part 61 includes a leading end portion 62, a fracturing portion 63, and an aspiration port 64. The outer diameter of the head part 61 is determined to be larger than the outer diameter of the shaft 65. The head part 61 has an almost constant outer diameter (slightly tapered) along the long axis direction. The head part 61 is designed with a sufficient length enough to ensure the fracturing portion 63 along the long axis direction and a length so that the leading end of the sleeve 70 is located near the head part 61.

The leading end portion 62 is formed at the front end (foremost end) of the head part 61. The leading end portion 62 has a shape less likely to damage the capsule when the leading end portion 62 touches the capsule of the lens. The leading end portion 62 has a rounded shape, i.e., a semi-spherical shape in the present embodiment. The fracturing portion 63 is formed at the base of the leading end portion 62. The outer diameter of the base portion of the leading end portion 62 is equal to the outer diameter of the fracturing portion 63. Thus, the leading end portion 62 is smoothly continuous to the fracturing portion 63, thereby reducing damage to the capsule even when the leading end side of the head portion 61 (the leading end portion 62 and the leading end side of the fracturing portion 63) touches the capsule.

The fracturing portion 63 is shaped to enable fracturing of the lens nucleus when the tip 60 is torsionally vibrated. As shown in FIG. 5, the cross sectional shape (the cross section in a direction perpendicular to the long axis) of the fracturing portion 63 is designed so that a radius (moving radius) from the turning center of the torsional vibration (coincident with the central axis of the head part 61) to the surface of the fracturing portion 63 is nonconstant or nonuniform in an circumference (over the outer periphery) of the fracturing portion 63. For instance, comparing a radius R1 from the center A to a point C (a corner C mentioned later) of the fracturing portion 63 and a radius R2 from a center A to a point P on the surface of the fracturing portion 63, a relationship of R1 > R2 is established.

Accordingly, when the fracturing portion 63 is torsionally vibrated (moved), a portion with the relatively long radius is vibrated, thereby fracturing a portion of the lens nucleus located nearby. In other words, the fracturing portion 63 is configured to have an irregular outer periphery (protrusions) with reference to the center of the torsional vibration.

To be concrete, the cross sectional shape of the fracturing portion 63 is formed in a shape having corners C (e.g., a regular polygon). In the present embodiment, a regular tetragon (square) is adopted. Since the aspiration port 64 is formed at one corner of the regular polygon, corners Ca are formed at the rim of the aspiration port 64. In a case where the cross sectional shape of the fracturing portion 63 is regular polygonal, less corners are preferable. If more corners are provided, the fracturing portion 63 has a more rounded shape, resulting in a decrease in fracturing effect by torsion. In a case where the cross sectional shape of the fracturing portion 63 is regular polygonal, it is preferable to choose from regular triangle to regular octagon.

Since the corners Ca are formed in the aspiration port 64, increasing the number of corner portions, the fracturing efficiency of the fracturing portion 63 by torsional vibration can be expected to increase. In particular, when the fracturing portion 63 is torsionally vibrated while the lens nucleus is adsorbed in the aspiration port 64 (occlusion of the aspiration port 64), the adsorbed lens nucleus is fractured more efficiently by the corners Ca.

Since the cross sectional shape of the fracturing portion 63 is polygonal, the fracturing portion 63 (the whole head part 61) can be designed with the outer diameter constant in the long axis direction. This can facilitate production of the tip 60.

The larger the outer diameter of the fracturing portion 63, the more fracturing efficiency of the lens nucleus can be enhanced. Accordingly, the outer diameter of the fracturing portion 63 is determined to be as large as possible. The outer diameter in this embodiment is defined by the radius from the center of torsional vibration to a farthest point on the surface (e.g., radius R1). To be concrete, the outer diameter of the fracturing portion 63 is set to be less than the outer diameter of the sleeve 70 but equal to or more than the outer diameter of the shaft 65 to avoid a large incision wound from being formed in a patient's eye. The outer diameter of the fracturing portion 63 is set to 1.0 mm or more and 3.0 mm or less. In the present embodiment, the outer diameter of the fracturing portion 63 is set to be approximately equal to the outer diameter of the sleeve 70. It is to be noted that a sufficient distance is ensured between a rear end of the head part 61 and the leading end of the sleeve 70 to avoid blockage of the irrigation passage.

The aspiration port 64 is a port (an opening) formed in the surface of the head part 61 to communicate with the aspiration passage 66. The aspiration port 64 is an inlet for aspirating the tissues of the lens fractured and the fluid, and others. The aspiration port 64 is formed in a position allowing easy aspiration of the tissues fractured by the fracturing portion 63 under ultrasonic vibration and a position allowing easy aspiration of residual cortical substances of the capsule and others while the head part 61 is not ultrasonically vibrated. Specifically, the aspiration port 64 is formed near the leading end portion 62 and the fracturing portion 63 to facilitate aspiration of the residual cortical substances peeled by the leading end portion 62 and others and a small lens nucleus, and aspiration of the lens nucleus fractured by the fracturing portion 63. The aspiration port 64 is preferably formed in the surface of the fracturing portion 63, on a side close to the leading end portion 62, i.e., in a front half of the fracturing portion 63. In the present embodiment, the aspiration port 64 is formed at a boundary (across the boundary) between the leading end portion 62 and the fracturing portion 63.

Cataract surgery using the tip configured as above will be explained below. Herein, the lens nucleus is fractured and cut by the ophthalmic laser surgical apparatus 200, and then the lens nucleus and others are removed by the ophthalmic surgical apparatus 100 and an IOL is injected.

Prior to the surgery, the operator makes settings of the apparatuses 100 and 200. Specifically, the operator sets variable parameters of ultrasonic vibration power (output and duty cycle) and others on the setting part 35. The operator further sets a parameter to fracture and cut the lens nucleus by operating the operation unit 250. Herein, a pattern to fracture the lens nucleus is set to a cube of 0.3 mm per side.

The operator operates the operation unit 250 to make fixation of the eyeball of the patient's eye E and alignment. The operator depresses the foot switch of the operation unit 250 to make laser irradiation. By this series of operations, the lens nucleus of the patient's eye E is cut by laser. Simultaneously, an anterior capsule of the lens is incised and an incision wound is formed.

The operator operates the apparatus 100. Specifically, the operator holds the handpiece 10 and inserts the tip 60 and the sleeve 70 into the patient's eye E. The operator operates the operation unit 36 to make aspiration and removal of the lens nucleus. At that time, an operation mode of the handpiece 10 by the operation unit 36 is an ultrasonic vibration mode. During aspiration, if the lens nucleus is too large to be aspirated through the aspiration port 64, the operator makes ultrasonic vibration of the tip 60. When the lens nucleus is fractured by the fracturing portion 63 under ultrasonic vibration, the thus fractured tissues are allowed to be aspirated through the aspiration port 64, and they are aspirated and removed. By repeating the above work, the lens nucleus in the capsule is removed.

After removal of the lens nucleus, the operator makes aspiration removal of residual cortical substances. Specifically, the operator operates the operation unit 36 to change the operation mode of the handpiece 10 to the irrigation aspiration mode. The operator moves the leading end portion 62 in contact with the capsule, thereby peeling the cortical substances remaining in the capsule. The operator makes an aspiration operation with the operation unit 36 and removes the peeled cortical substances by aspiration. At that time, the operator removes the cortical substances efficiently by adsorbing the cortical substances in the aspiration port 64. By repeating this work, the tissues in the lens are removed. After the removal work, the operator places an IOL in the capsule. Thus, the cataract surgery is completed.

In the above cataract surgery, the single tip 60 attached to the handpiece 10 enables aspiration removal of the lens nucleus and the removal of the residual cortical substances. At that time, the lens nucleus has already been fractured and incised by the apparatus 200, it is easily aspirated and removed. In a case where the lens nucleus is larger than the aspiration port 64, for instance, in a case where the lens nucleus could not be sufficiently fractured and incised by the apparatus 200, the tip 60 is torsionally vibrated, so that another lens nucleus can be fractured and removed by aspiration. Cases where the lens nucleus could not be fractured and incised by the apparatus 200 are a site (e.g., lens equator) to which a laser beam does not reach and a site (near the posterior capsule) to which no laser is irradiated to reduce risks of breakage of the capsule. These sites are peripheral portions of the lens and the lens nucleus is relatively soft. Accordingly, even the fracturing portion 63 as configured in the present embodiment can fracture the lens nucleus sufficiently. During aspiration removal of the residual cortical substances, the leading end portion 62 being rounded is less likely to damage the capsule even when the leading end portion 62 touches the capsule. The leading end portion 62 is also able to abrade the capsule (peel the residual cortical substances).

In the above manner, the aspiration removal of the lens nucleus and the aspiration removal of the residual cortical substances can be performed by the single tip 60. This eliminates the need to replace the handpiece (tip) for aspiration removal of the lens nucleus and the handpiece (tip) for aspiration removal of the residual cortical substances during surgery, i.e., the replacement time and work can be saved. This can shorten the operative duration of time and reduce loads on a patient. Furthermore, it is unnecessary to insert another tip (a second tip) into the patient's eye, resulting in a decrease in risk of infectious disease (e.g., germs on the surface of a patient's eye enter the eye).

In the above explanations, the cross sectional shape of the fracturing portion 63 is regular tetragonal, but it is not limited thereto. The fracturing portion 63 is required only to have a corner serving as a protrusion. The cross sectional shape of the fracturing portion 63 may be selected from tetragon (rectangle), rhombus, parallelogram, trapezium, and other shapes. Other shapes may be star-shape, semi-circular (segmented circular) shape, cross shape, etc. The outer diameter of the fracturing portion 63 may not be constant (uniform cross sectional shape) along the long axis direction. For instance, the aforementioned cross sectional shape may be tapered toward the leading end or toward the rear end. The fracturing portion 63 also may be formed with external threads on the surface, a groove extending along the long axis direction, or pearskin finish.

In the above explanations, the outer diameter of the head part 61 is set to be equal to or less than the outer diameter of the sleeve 70, but it is not limited thereto. The head part 61 is required only to be smaller than an incision wound formed in a patient's eye. In other words, the outer peripheral length of the head part 61 has only to be equal to or less than the circumferential length of the incision wound. The outer diameter of the head part 61 may be larger than the outer diameter of the sleeve 70. The sleeve 70 may be formed in a tapered shape. In this case, the outer diameter of the head part 61 is likely designed to be larger than the outer diameter of the leading end (an opening) of the sleeve 70.

In the above explanations, the leading end portion 62 and the fracturing portion 63 are smoothly continuous to each other, but the invention is not limited thereto. A step or shoulder may also be provided between the leading end portion 62 (on the proximal end side) and the fracturing portion 63.

In the above explanations, the aspiration port 64 is formed at the corner of the fracturing portion 63, but it is not limited thereto. The aspiration port 64 may be formed in any side surface of the fracturing portion 63, e.g., in a rectangular surface as long as it is able to aspirate the fractured tissues and others.

In the above explanations, the aspiration port 64 is formed near the boundary between the leading end portion 62 and the fracturing portion 63, but it is not limited thereto. The aspiration port 64 may be formed in any position that can efficiently aspirate the tissues fractured by the fracturing portion 63 during ultrasonic vibration of the tip 60 and that can efficiently remove and aspirate the residual cortical substances by the head part 61 when the tip 60 is used only for aspiration. For instance, the aspiration port may be formed in a proximal end portion or in a leading end portion of the fracturing portion.

The above explanation shows that the aspiration port 64 is formed in a circular shape, but not limited thereto. The aspiration port 64 may be formed in any shape as long as it can be occluded by the tissues of a lens but it does not aspirate the tissues into the port. The aspiration port may be shaped in oval, slit-like, etc. Further, a plurality of aspiration ports may be formed.

The above explanations exemplify the apparatus using a femtosecond laser beam as a second apparatus different from the apparatus 100 to make incision of the lens nucleus and others. However, the invention is not limited thereto. The second apparatus may have any configuration if only it is able to incise the lens nucleus and others. For example, it may be another laser surgical apparatus, surgical scissors, catheter, or the like.

The above explanations are mere examples and do not any limitations to the present invention. The present invention may be embodied in other specific forms without departing from the essential characteristics thereof.

### Reference Sings List

- 3: Ultrasonic transducer
- 5: Horn
- 10: Handpiece
- 13: Irrigation tube
- 60: Tip
- 61: Head part
- 62: Leading end portion
- 63: Fracturing portion
- 64: Aspiration port
- 65: Shaft
- 70: Sleeve
- 100: Ophthalmic surgical apparatus
- 200: Ophthalmic laser surgical apparatus

## Claims

1. An ophthalmic surgical tip (60) which will be attached to an ophthalmic surgical handpiece (10) including an ultrasonic transducer (3), the tip being configured to fracture a lens nucleus by utilizing torsional vibration from the ultrasonic transducer, the tip including a small tube internally formed with an aspiration passage (66), and the tip being arranged to aspirate and remove fractured tissues through the aspiration passage,
**characterized by** including:
a hollow shaft (65) in which the small tube is formed; and
a head part (61) formed on a leading end side of the shaft, the head part including: a leading end portion (62) formed in a rounded shape at a leading end of the head part; a fracturing portion (63) formed behind the leading end portion and having a protrusion to fracture a lens nucleus by torsional vibration; and an aspiration port (64) connected to the small tube.

2. The ophthalmic surgical tip according to claim 1, wherein the leading end portion is semi-spherical.

3. The ophthalmic surgical tip according to claim 1 or 2, wherein the fracturing portion has a cross sectional shape in a direction perpendicular to an axis direction such that a radius (R1, R2) from a turning center (A) of torsional vibration of the fracturing portion to a surface of the fracturing portion is nonconstant in a circumferential direction of the fracturing portion.

4. The ophthalmic surgical tip according to any one of claims 1 to 3, wherein the head part has an outer diameter equal to or larger than the shaft and equal to or less than an outer diameter of a sleeve (70) covering the shaft.

5. The ophthalmic surgical tip according to any one of claims 1 to 4, wherein the cross sectional shape of the fracturing portion in the direction perpendicular to the axis direction is polygonal.

6. The ophthalmic surgical tip according to any one of claims 1 to 5, wherein the outer diameter of the fracturing portion is 1.0 mm or more and 3.0 mm or less.

7. The ophthalmic surgical tip according to any one of claims 1 to 6, wherein the aspiration port has a circular shape having a diameter of 0.3 mm or more and 0.5 mm or less.

8. The ophthalmic surgical tip according to any one of claims 1 to 7, wherein the aspiration port is formed in the fracturing portion.

9. An ophthalmic surgical handpiece including the ophthalmic surgical tip set forth in any one of claims 1 to 8.

10. An ophthalmic surgical apparatus including the ophthalmic surgical handpiece set forth in claim 9.
